(19) Europäisches Patentamt
European Patent Office
Office européen des brevets

(11) Veröffentlichungsnummer : **0 126 944 B2**

# (12) NEUE EUROPÄISCHE PATENTSCHRIFT

(45) Veröffentlichungstag der neuen Patentschrift : **26.08.92 Patentblatt 92/35**

(51) Int. Cl.$^5$ : **A61K 7/00,** A61K 7/32

(21) Anmeldenummer : **84104314.4**

(22) Anmeldetag : **16.04.84**

## (54) Verwendung von 6-(Z)- bzw. 2-(Z)-konfigurierten 3,7,11-Trimethyl-dodeca-2,6,10-trien-1-olen als Bakteriostatikum in kosmetischen Produkten.

Die Akte enthält technische Angaben, die nach dem Eingang der Anmeldung eingereicht wurden und die nicht in dieser Patentschrift enthalten sind.

(30) Priorität : **26.04.83 DE 3315058**

(43) Veröffentlichungstag der Anmeldung : **05.12.84 Patentblatt 84/49**

(45) Bekanntmachung des Hinweises auf die Patenterteilung : **04.10.89 Patentblatt 89/40**

(45) Bekanntmachung des Hinweises auf die Entscheidung über den Einspruch : **26.08.92 Patentblatt 92/35**

(84) Benannte Vertragsstaaten : **AT CH DE FR GB LI NL**

(56) Entgegenhaltungen :
**DE-A- 2 728 921**
**JP-A- 7 891 122**

(56) Entgegenhaltungen :
**CHEMICAL ABSTRACTS, Band 90, 1979, Seite 185, Ref.Nr. 17664a, Columbus, Ohio, US; & JP - A - 78 91 122 (KURARAY CO.,LTD.) 10.08.1978**
**CHEMICAL ABSTRACTS, 1977-1981, Chem. Substance Index, Seite 19381, Columbus, Ohio, US**
**JOURNAL OF ORGANIC CHEMISTRY, Band 26, 1963, Seiten 1086-1089, American Chemical Society, Easton, US; BATES et al.: "Thestereoisomeric farnesols"**

(73) Patentinhaber : **Dragoco Gerberding & Co. GmbH**
**Dragocostrasse 1**
**W-3450 Holzminden (DE)**

(72) Erfinder : **Brunke, Ernst-Joachim**
**Holbeinstrasse 6**
**W-3450 Holzminden (DE)**
Erfinder : **Klein, Erich**
**Wiesenweg 50**
**W-3450 Holzminden (DE)**

(74) Vertreter : **Patentanwälte Deufel, Hertel, Lewald**
**Postfach 26 02 47 Isartorplatz 6**
**W-8000 München 26 (DE)**

EP 0 126 944 B2

## Beschreibung

Die menschliche Hautflora besteht im Normalzustand aus einem Gleichgewicht verschiedener Mikroorganismen, die zumeist keine negativen Auswirkungen für Gesundheit und Wohlbefinden des Menschen haben. Durch bakteriellen Abbau verschiedener nicht oder nur schwach riechender Bestandteile des Körperschweißes werden oft stark riechende Verbindungen gebildet, die vom jeweiligen Menschen selber oder auch seiner Umwelt als unangenehm und störend empfunden werden. Als kosmetische Mittel, die diese Art der Ausbildung von Körpergeruch unterbinden, sind Antiperspirants im Markt, die die Schweißabsonderung unterdrücken, oder Deodorants, die durch Bakterizide mit drastischer Wirkung nahezu die gesamte Hautflora lokal zerstören. Derartige baktericide Wirkstoffe sind meistens halogenhaltige Phenolderivate.

Mit der Absicht, einen natürlichen Wirkstoff ohne toxikologisches Risiko bereitzustellen, beschreibt die DE-A- 2 728 921 die Verwendung des Sesquiterpenalkohols Farnesol (2) als ein Bakteriostatikum, das das Wachstum der geruchsbildenden Teile der Hautflora unterbindet, ohne das biologische Gleichgewicht der Haut wesentlich zu verändern. Farnesol (2), das 2-trans, 6-transkonfigurierte 3, 7, 11-Trimethyl-dodeca-2, 6, 10-trien-1-ol, ist in der Natur weit verbreitet und als Inhaltsstoff vieler ätherischer Öle bekannt (Merkel, D. in: "Die Atherischen Öle" (Herausg.: W. Treibs) Vol. III6, Akademie Verlag, Berlin 1962). Die bakteriostatische Wirkung des Naturstoffs kam erst nach Anreicherung auf eine bakteriostatisch wirksame Konzentration zum fragen und konnte erst dann erkannt werden (DE-A-2 728 921).

Aus Journal of Organic Chemistry, Bd. 28, 1963, Seiten 1086 - 1089, ist es bekannt, daß natürliches trans-/trans- Farnesol auch im Gemisch mit dem in vorliegender Patentschrift als (1a) bezeichneten cis-trans-Isomer vorkommen kann, so daß nicht auszuschließen ist, daß bei den in obiger DE-A-2 728 921 beschriebenen Arbeiten bereits ein Gemisch dieser beiden Isomere in Kosmetischen Mitteln zum Einsatz gelangte.

Die begrenzte Verfügbarkeit bestimmter Naturprodukte und die relativ hohen Kosten für die Gewinnungreiner Wirkstoffe aus ätherischen Ölen oder Pflanzen-Extrakten schränken die industrielle Anwendung dieser Wirkstoffe ein. Es ist daher anzustreben, den Naturstoff oder strukturell verwandte Verbindungen mit ähnlicher oder sogar verbesserter Wirkung und ähnlich geringen Nebenwirkungen durch chemische Synthese darzustellen.

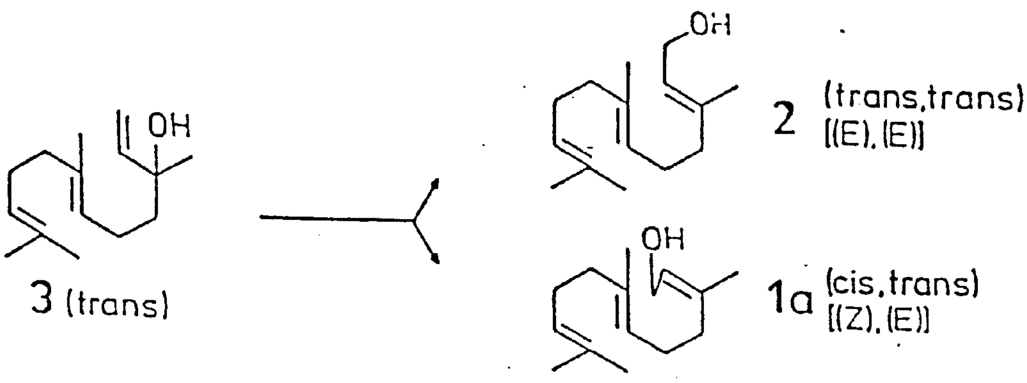

3 (trans)

2 (trans,trans) [(E),(E)]

1a (cis,trans) [(Z),(E)]

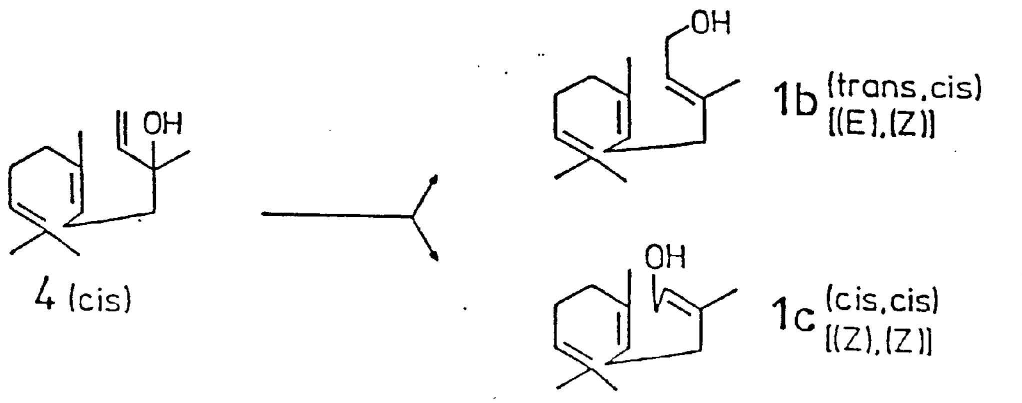

4 (cis)

1b (trans,cis) [(E),(Z)]

1c (cis,cis) [(Z),(Z)]

In der Natur wurde bisher hauptsächlich Farnesol (2) mit 2-trans,6-trans-Geometrie gefunden und als dessen Vorläufer das trans-Nerolidol (3) (Y. - R. Naves, Comptes rendus hebdomadaires des séances de l'Academie des Siences, 251, 900 (1968)). Die isomeren Sesquiterpenalkohole trans-Nerolidol (3) und cis-Nerolidol (4) sind durch Tolalsynthese jeweils in hoher Reinheil zugänglich (V. Herout in Fragrance Chemistry (Herausgeber: E. Theimer), S. 226, Academic Press, New York 1982). Bei der in vitro-Allylumlagerung von trans-Nerolidol (3) (L. Ruzicka, Helv. Chim. Acta 6, 483 (1923)) entsleht ein Gemisch von 2 und dem 2-cis, 6-trans-Isomeren 1a. Aus cis-Nerolidol (4) entsteht bei der Allylumlagerung ein Gemisch der isomeren 1b und 1c. Die geometrischen Isomeren 1b und 1c sind bisher nicht als Naturprodukte, bzw. Bestandteil ätherischer Öle nachgewiesen worden. Aus einem technisch produzierbaren Gemisch der isomeren Nerolidole 3 und 4 wird durch Allylumlagerung ein Gemisch der 4 geometrischen isomeren 2, 1a, 1b und 1c gebildel. Durch fraktionierte Destillation lassen sich Farnesol (2) und 1a sowie die nicht natürlichen Isomere 1b - c rein darstellen (R.D. Bates, D.M. Gale und B.J. Grunar, J. org. Chem. 28, 1086 (1963)).

Aus der japanischen Offenlegungsschrift 78/91122 sind Mittel gegen Mikroorganismen bekannt, die nicht hautreizend sind und sich für kosmetische Zwecke eignen. Diese Mittel enthalten als aktive Substanzen verschiedene Terpen-Alkohole, insbesondere trans-/trans-Farnesol (Verbindung 2) und dessen 2-trans/6-cis-Isomeres (Verbindung 1b).

Es wurde gefunden, daß die aus den Synthese-Gemischen 1a/2,1b/1c oder 2+1a-c durch Destillation gewonnene Verbindung 1c im Vergleich zu den Verbindungen 1a und 2 wirksamer ist und daß unter den bisher gewählten Versuchsbedingungen die Verbindung 1b eine größere Wirksamkeit aufweist als dies der bisherige Stand der Technik erwarten ließ.

Auch das im technischen Maßstab zu erzeugende Synthese-Gemisch der cis-cis-Verbindung (1c) im Gemisch mit den anderen Isomeren läßt sich vorteilhaft als naturanaloge Desodorans verwenden.

Die mikrobiologischen Prüfungen wurden mit den Bakterienarten Staphylococcus aureus, Corynebacterium species und Staphylococcus epidermidis durchgeführt. Es wurden jeweils Konzentrationen von 0,3 % der Verbindungen 1a, b, c, 2 gegen unterschiedliche Keimzahlen eingesetzt und 1. mit einer Aufschwemmung von $10^4$ koloniebildenden Einheiten/ml (KBE/ml) und 2. mit $10^6$ KBE/ml versehen. Die Durchführung der Untersuchung erfolgte in üblicher Weise, indem Filterpapierplättchen mit einer Fläche von 15,9 $cm^2$ mit je 0,2 ml der 0,3 % igen alkoholischen Konzentrationen beschickt wurden. Nach Trocknen der aufgebrachten Lösungen wurden die Filterpapierplättchen dann in den Agar von Petrischalen eingebettet, so daß die Oberfläche mit einer dünnen Agarschicht überschichtet werden konnte. Danach wurde die ganze Platte mit den Testbakterien in unterschiedlicher Keimkonzentration beimpft (Tabelle 1)

Für die Verbindungen 1a, 1b, 1c und das Gemisch 2 + 1a - c läßt sich eine deutliche antibakterielle Wirkung gegenüber allen drei geprüften Bakterienaren nachweisen. Nach dem Testergebnis zeigen die Verbindungen 1a, b, c die besten Wirksamkeiten, die auch bei Slaphylococcus epidermidis eine fast totale Hemmung nach sich gezogen haben. Das natürliche Farnesol (2) ist dagegen sowohl gegenüber Staphylococcus epidermidis als auch gegenüber Staphylococcus aureus von etwas geringerer Wirksamkeit. Alle Formulierungen haben bei der vorgegebenen Keimzahl gegenüber Corynebacterium spec. eine tolale Vermehrungshemmung nach sich gezogen, was für die Verhinderung von Körpergeruch von größerer Bedeutung ist als die Hemmung von Staphylococcus epidermidis.

**Tabelle 1:**

Vertikaler Diffusionstest, durchgeführt mit den geometrischen Isomeren des Farnesols und dem synthetischen Farnesolgemisch gegenüber Corynebacterium species, Staphylococcus epidermidis und Staphylococcus aureus SG 511 bei unterschiedlichen Keimzahlen (KBE/ml)

| Testsubstanz | Konzentration | Anwendung | Corynebacteriumt species | | Staphylococcus epidermidis | | Staphylococcus aureus SG 511 | |
|---|---|---|---|---|---|---|---|---|
| | | | $10^4$ | $10^6$ KBE/ml | $10^4$ | $10^6$ KBE/ml | $10^4$ | $10^6$ KBE/ml |
| Substanz 1a (cis, trans) | 0,3 % | 0,2 ml/ 15,9 $cm^2$ | 0 | 0 | 0 - 1 | 0 - 1 | 0 | 0 |
| Substanz 1b (trans, cis) | 0,3 % | 0,2 ml/ 15,9 $cm^2$ | 0 | 0 | 0 | 0 - 1 | 0 | 0 |
| Substanz 1c (cis, cis) | 0,3 % | 0,2 ml/ 15,9 $cm^2$ | 0 | 0 | 0 | 0 - 1 | 0 | 0 |
| Substanz 2 (trans-trans) | 0,3 % | 0,2 ml/ 15,9 $cm^2$ | 0 | 0 | 1 - 2 | 1 - 2 | 0 - 1 | 0 - 1 |
| Gemisch 2 + 1a + 1b + 1c | 0,3 % | 0,2 ml/ 15,9 $cm^2$ | 0 | 0 | 0 - 1 | 1 - 2 | 0 | 0 |
| Ethylalkohol als Kontrolle | 96 % | 0,2 ml/ 15,9 $cm^2$ | 4 | 4 | 4 | 4 | 4 | 4 |

## Patentansprüche

1. Verwendung von 6-(Z)- und 2-(Z)-konfiguriertem 3,7,11-Trimethyl-dodeca-2,6,10-trien-1-ol der Formel 1c

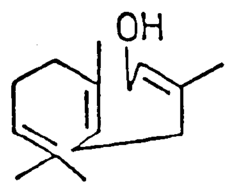

1c (cis,cis) [(Z),(Z)]

als Bakteriostatikum in kosmetischen Mitteln zu Schutz und Pflege der menschlichen Haut.

2. Verwendung der Verbindung nach Anspruch 1 im Gemisch mit der 2-cis-6-trans-, der 2-trans-6-cis-Verbindung, gegebenenfalls auch in Kombination mit der 2-trans-6-trans-Verbindung.

3. Kosmetische Mittel zur Desodorierung menschlicher Hautpartien, gekennzeichnet durch einen bakteriostatisch wirksamen Gehalt von mindestens 0,15 % an 3,7,11-Trimethyl-dodeca-2,6,10-trien-1-olen nach Anspruch 1 oder dem Gemisch nach Anspruch 2.

## Revendications

1. Utilisation du 3,7,11-triméthyldodéca-2,6,10-triène-1-ol à configuration 6-(Z) et 2-(Z), de formule 1c

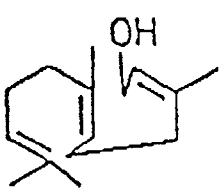

1c (cis,cis) [(Z),(Z)]

comme bactériostatique dans des compositions cosmétiques pour la protection et les soins de la peau humaine.

2. Utilisation du composé suivant la revendication 1 en mélange avec le composé 2-cis-6-trans, le composé 2-trans-6-cis, le cas échéant également en association avec le composé 2-trans-6-trans.

3. Compositions cosmétiques pour supprimer les odeurs de parties de la peau humaine, caractérisées par une teneur à effet bactériostatique d'au moins 0,15 % de 3,7,11-triméthyldodéca-2,6,10-triène-1-ols suivant la revendication 1 ou de mélange suivant la revendication 2.

## Claims

1. Use of 6-(Z)- and 2-(Z)-configurated 3,7,11-trimethyl-dodeca-2,6,10-trien-1-ol of the formula 1c

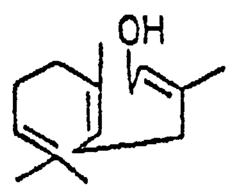

1c (cis, cis) [(Z), (Z)]

as a bacteriostat in cosmetic agents for the protection and care of the human skin.

2. Use of the compound according to Claim 1 as a mixture with the 2-cis-6-trans, the 2-trans-6-cis compound, if appropriate also as a combination with the 2-trans-6-trans compound.

3. Cosmetics for deodorising areas of the human skin, characterised in that they contain a bacteriostatically active content of at least 0.15% 3,7,11-trimethyldodeca-2,6,10-trien-1-ols according to Claim 1, or of the mixture according to Claim 2.